# EUROPEAN PATENT APPLICATION

(11) **EP 4 000 393 A1**
(43) Date of publication of application: **25.05.2022**
(21) Application number: 20839992.3
(22) Date of filing: 14.07.2020
(51) Int. Cl.: A01K 63/04

(54) **AQUACULTURE SYSTEM**

(30) Priority: 16.07.2019 JP 2019131003
(71) Applicant: NGK SPARK PLUG CO., LTD., Nagoya-shi, Aichi 467-8525 (JP)
(72) Inventor: TAKEHIRO, Yoji, Nagoya-shi, Aichi 467-8525 (JP); TAMAI, Kazusei, Nagoya-shi, Aichi 467-8525 (JP); SAITO, Miyuki, Nagoya-shi, Aichi 467-8525 (JP); PUTRA, Gray Lawrence Sirosi, Nagoya-shi, Aichi 467-8525 (JP); TAKENOSHITA, Koyo, Tokyo 105-0001 (JP); YOSHIDA, Isamu, Tokyo 105-0001 (JP); NISHIMURA, Ryoji, Tokyo 105-0001 (JP); YAMASAKI, Yusuke, Tokyo 105-0001 (JP)
(74) Representative: Grünecker Patent- und Rechtsanwälte PartG mbB
(86) International application number: PCT/JP2020/027385
(87) International publication number: WO 2021/010399

(57) **Abstract**

This aquaculture system (Sy) comprises: mineral sensors (22, 24, 26) that detect the concentration of minerals contained in breeding water in a breeding tank (50) for breeding an aquatic organism or in breeding water in a circulation route (70); and an adjustment part that performs an instruction or an action for inducing the concentration of the minerals in the breeding water to coincide with the standard when the mineral concentration detected by the mineral sensors (22, 24, 26) is out of the standard.

## Description

### TECHNICAL FIELD

The present invention relates to an aquaculture system.

### BACKGROUND ART

Patent Literature 1 discloses a shrimp raising and health tending system to be used in indoor shrimp production. Rearing water which is used in a shrimp plant to which this system is applied contains sodium chloride, magnesium chloride, calcium chloride, sodium bicarbonate, sodium sulfate, and potassium chloride. Patent Literature 1 states that calcium and magnesium are necessary for molting and growth of shrimps.

Also, necessity of minerals for rearing not only shrimps but also various other aquatic organisms is generally known.

### CITATION LIST

### PATENT LITERATURE

Patent Literature 1: Japanese Patent Application Laid-Open (kokai) No. 2008-43252

### SUMMARY OF INVENTION

### TECHNICAL PROBLEM

Some aquatic organism culturing methods provided or proposed heretofore use mineral-containing rearing water for rearing an aquatic organism. However, the mineral concentration of rearing water is not adjusted or controlled in some way so as to render the mineral concentration coincident with its standard. Minerals are components that are consumed with growth of the aquatic organism. Therefore, the concentrations of the minerals gradually decrease from those at the time when rearing is started or those at the time of water exchange. However, adjusting the minerals contained in the rearing water during rearing has been difficult, because the amounts of minerals which are to be added vary depending on the current water amount and the current mineral concentrations.

The present invention has been accomplished so as to solve at least part of the above-described problem and its object is to provide an aquaculture system which can adjust the concentration of a mineral contained in rearing water stored in a rearing tank so that the mineral concentration coincides with its standard.

### SOLUTION TO PROBLEM

An aquaculture system which is one solution of the present invention is a recirculating-type aquaculture system for culturing an aquatic organism, comprising:
a mineral sensor for detecting the concentration of a mineral which is contained in rearing water within a rearing tank for rearing the aquatic organism or the rearing water within a circulation passage through which the rearing water circulates outside the rearing tank; and
an adjustment section which compares the concentration of the mineral detected by the mineral sensor with a predetermined standard and performs instruction issuance or operation for rendering the concentration of the mineral contained in the rearing water coincident with the standard when the mineral concentration deviates from the standard.

In the above-described aquaculture system, the concentration of a mineral which is contained in rearing water within the rearing tank or the rearing water within the circulation passage can be detected by the mineral sensor, and when the mineral concentration detected by the mineral sensor deviates from the standard, the instruction issuance or operation for rendering the concentration of the mineral coincident with the standard can be performed by the adjustment section. Therefore, it is possible to optimize the concentration of the mineral contained in the rearing water, thereby preventing the aquaculture system from being operated continuously in a state in which the concentration of the mineral has deviated from the standard.

Herein, the term "standard" refers to a predetermined numerical value or numerical range which can be set on the basis of well-known knowledges such as an optimum value for growth of an aquatic organism and the fact that deviation of the concentration of the mineral from the standard adversely affects the growth of the aquatic organism.

In the above-described aquaculture system, the adjustment section may perform instruction issuance or operation for adding the mineral to the rearing water when the mineral concentration detected by the mineral sensor is lower than the standard.

In this aquaculture system, when the mineral concentration of the rearing water is lower than the standard; namely, when the mineral is insufficient, instruction issuance or operation for making up for the insufficiency can be performed by the adjustment section. Accordingly, it is possible to prevent the aquaculture system from being operated continuously in a state in which the mineral concentration of the rearing water is excessively low, thereby restraining occurrence of a problem due to excessive continuation of a period during which the mineral concentration of the rearing water is excessively low.

In the above-described aquaculture system, the adjustment section may perform instruction issuance or operation for adding water into the rearing tank or to the circulation passage when the mineral concentration detected by the mineral sensor is higher than the standard.

In this aquaculture system, when the mineral concentration of the rearing water within the rearing tank is higher than the standard; namely, when the mineral is excessive, instruction issuance or operation for remedying the excessive state can be performed by the adjustment section. Accordingly, it is possible to prevent the aquaculture system from being operated continuously in a state in which the mineral concentration of the rearing water is excessively high, thereby restraining occurrence of a problem due to excessive continuation of a period during which the mineral concentration of the rearing water is excessively high.

In the above-described aquaculture system, the adjustment section may perform operation for issuing a warning to the outside when the mineral concentration detected by the mineral sensor deviates from the standard.

Since this aquaculture system can perform the operation for issuing a warning to the outside when the mineral concentration of the rearing water deviates from the standard, an operator or the like who has recognized the warning operation can grasp the anomaly of the mineral concentration. Therefore, the operator can quickly take a countermeasure suitable for the case where the mineral concentration of the rearing water deviates from the standard.

In the above-described aquaculture system, the aquatic organism to be reared may be a crustacean.

In the case where a crustacean is reared in the rearing water, the concentration of the mineral contained in the rearing water is more important, and, when the period during which the mineral concentration is not proper becomes excessively long, the risk that the crustacean is not reared properly becomes higher. In this regard, the above-described aquaculture system can optimize the mineral concentration of the rearing water and it is possible to prevent the aquaculture system from being operated continuously in a state in which the mineral concentration has deviated from the standard. Therefore, it is possible to reduce the risk that the crustacean is not grown properly and to stabilize and accelerate the growth of the crustacean.

In the above-described aquaculture system, the above-described mineral sensor may be a liquid membrane type mineral sensor.

Since the liquid membrane type sensor can be easily reduced in size and does not require a special pre-processing or can reduce the amount of the special pre-processing, measurement can be performed easily.

The above-described aquaculture system may comprise mineral sensors for detecting the concentrations of a plurality of kinds of minerals. Also, the above-described adjustment section may perform instruction issuance or operation for rendering the respective concentrations of the plurality of kinds of minerals coincident with their standards.

This aquaculture system can easily adjust the respective concentrations of the plurality of kinds of minerals such that respective concentrations coincide with their standards and can be easily operated in a state in which the respective concentrations of the plurality of kinds of minerals are optimized.

In the above-described aquaculture system, in the case where the above-described adjustment section performs instruction issuance or operation for adding the above-described two or more kinds of minerals to the above-described rearing water, the above-described adjustment section may calculate the respective addition amounts of the above-described two or more kinds of minerals to be added and perform instruction issuance or operation for adding to the above-described rearing water the above-described two or more kinds of minerals mixed together on the basis of the above-described respective addition amounts.

When two or more kinds of minerals are added, if the minerals are added one kind by one kind, a period of time during which the minerals are in poor balance may occur. Since the above-described aquaculture system can mix the two or more kinds of minerals properly and add them at the same time, it is possible to easily prevent occurrence of a period of time during which the minerals are in poor balance.

In the above-described aquaculture system, in the case where the above-described adjustment section performs instruction issuance or operation for adding water to the above-described rearing water, before performance of the instruction issuance or operation for adding water to the above-described rearing water, the above-described adjustment section may calculate a necessary water supply amount and predict changes in the concentrations of the above-described plurality of kinds of minerals as a result of addition of water of the above-described water supply amount. When the concentration of at least one of the above-described plurality of kinds of minerals is predicted to become lower than its standard, the above-described adjustment section may calculate a mineral supply amount necessary to bring the concentration of the above-described at least one kind of mineral to the above-described standard and perform instruction issuance or operation for adding water on the basis of the above-described calculated water supply amount and instruction issuance or operation for adding the above-described at least one kind of mineral on the basis of the above-described mineral supply amount. When it is predicted that none of the above-described plurality of kinds of minerals becomes lower in concentration than the above-described standard, the adjustment section may perform instruction issuance or operation for adding water on the basis of the above-described calculated water supply amount.

In the case where water is added to the rearing water, the above-described aquaculture system can predict insufficiency of a particular mineral caused by the addition of water and can make up for the insufficiency.

### ADVANTAGEOUS EFFECTS OF INVENTION

The present invention can adjust the concentration of a mineral contained in rearing water stored in the rearing tank so that the mineral concentration coincides with its standard.

### BRIEF DESCRIPTION OF DRAWINGS

[FIG. 1] FIG. 1 is an explanatory diagram used for briefly describing an aquaculture system of a first embodiment.
[FIG. 2] FIG. 2 is a block diagram schematically illustrating the electrical configuration of the aquaculture system of FIG. 1.
[FIG. 3] FIG. 3 is an explanatory diagram specifically illustrating a portion of the aquaculture system of FIG. 1.
[FIG. 4] FIG. 4 is a flowchart illustrating the flow of mineral adjustment control performed in the aquaculture system of FIG. 1.
[FIG. 5] FIG. 5 is a flowchart illustrating the flow of mineral adjustment control performed in an aquaculture system of a second embodiment.

### DESCRIPTION OF EMBODIMENTS

### <First embodiment

### 1. Outline of aquaculture system

A first embodiment will be described with reference to the drawings.

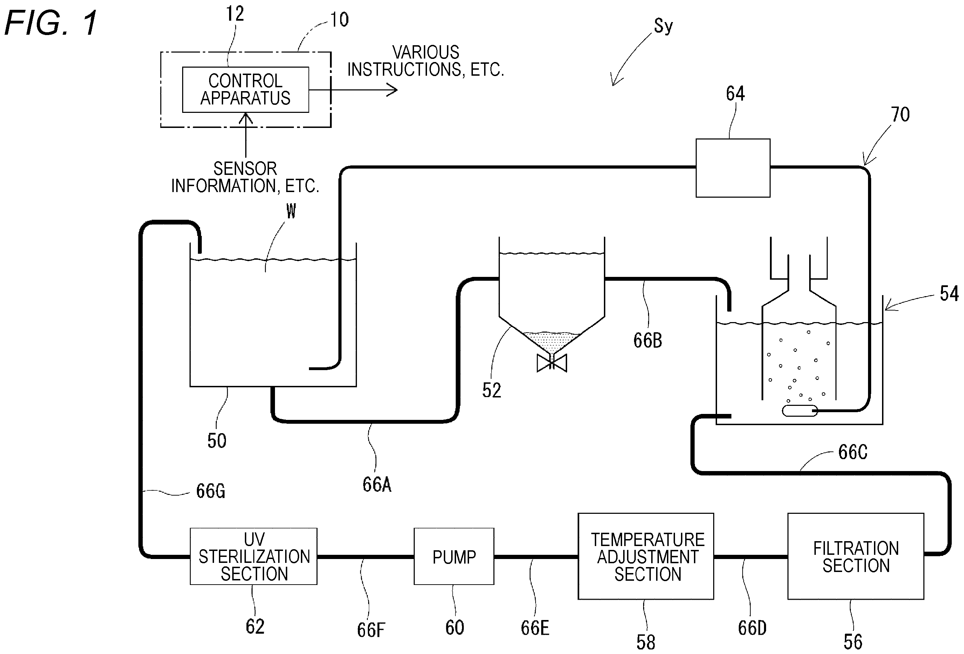

An aquaculture system Sy shown in FIG. 1 is a system for rearing and culturing an aquatic organism and is provided at a production base where the aquatic organism is reared and cultured. The following is description of an example in which the aquaculture system Sy is configured in the form of a recirculating-type land-based aquaculture system. Also, in the following description, a crustacean such as shrimp or prawn is shown as an example of the aquatic organism, and an example in which the crustacean is cultured by the aquaculture system Sy will be described. Notably, the aquaculture system Sy may be a closed-recirculating-type land-based aquaculture system in which the entirety or approximately entirety of rearing water discharged from a rearing tank 50 is circulated and returned to the rearing tank 50 or a semi-closed-recirculating-type land-based aquaculture system in which part of the rearing water discharged from the rearing tank 50 is circulated and returned to the rearing tank 50.

The aquaculture system Sy shown in FIG. 1 includes an information processing apparatus 10, the rearing tank 50, a sedimentation tank 52, a bubble separation tank 54, a filtration section 56, a temperature adjustment section 58, a pump 60, a UV sterilization section 62, an oxygen supply apparatus 64, pipe lines 66A to 66G, etc.

The rearing tank 50 is a water tank in which rearing water is stored and an aquatic organism is reared. The sedimentation tank 52 is a tank in which solid substances contained in the rearing water are sedimented for solid liquid separation. The bubble separation tank 54 is a layer in which bubbles (air bubbles) are generated by a bubble separation apparatus and contaminants in the rearing water are caused to adhere to the bubbles for separation. The filtration section 56 is a facility for filtering the rearing water and includes, for example, a tank which enables successive performance of physical filtration and biological filtration. The temperature adjustment section 58 is a facility for adjusting the temperature of the rearing water and is configured, for example, in the form of a tank which can heat and cool the rearing water. The pump 60 is a pump for circulating the rearing water. The UV sterilization section 62 is a facility for sterilizing the flowing rearing water by using a UV germicidal lamp. The oxygen supply apparatus 64 is an apparatus for supplying oxygen into the rearing tank 50 so as to optimize the amount of oxygen dissolved in the rearing water.

In the system of FIG. 1, a pipe line 66A for leading the rearing water within the rearing tank 50 to the sedimentation tank 52 is provided between the rearing tank 50 and the sedimentation tank 52. A pipe line 66B for leading the rearing water within the sedimentation tank 52 to the bubble separation tank 54 is provided between the sedimentation tank 52 and the bubble separation tank 54. A pipe line 66C for leading the rearing water within the bubble separation tank 54 to the filtration section 56 is provided between the bubble separation tank 54 and the filtration section 56. A pipe line 66D for leading the rearing water having passed through the filtration section 56 to the temperature adjustment section 58 is provided between the filtration section 56 and the temperature adjustment section 58. A pipe line 66E for leading the rearing water having passed through the temperature adjustment section 58 to the pump 60 is provided between the temperature adjustment section 58 and the pump 60. A pipe line 66F for leading the rearing water having passed through the pump 60 to the UV sterilization section 62 is provided between the pump 60 and the UV sterilization section 62. A pipe line 66G for leading the rearing water having passed through the UV sterilization section 62 to the rearing tank 50 is provided between the UV sterilization section 62 and the rearing tank 50. A circulation passage 70 is formed by the sedimentation tank 52, the bubble separation tank 54, the filtration section 56, the temperature adjustment section 58, the pump 60, the UV sterilization section 62, and the pipe lines 66A, 66B, 66C, 66D, 66E, 66F, and 66G. The circulation passage 70 is a passage through which the rearing water within the rearing tank 50 circulates outside the rearing tank 50 and returns to the rearing tank 50.

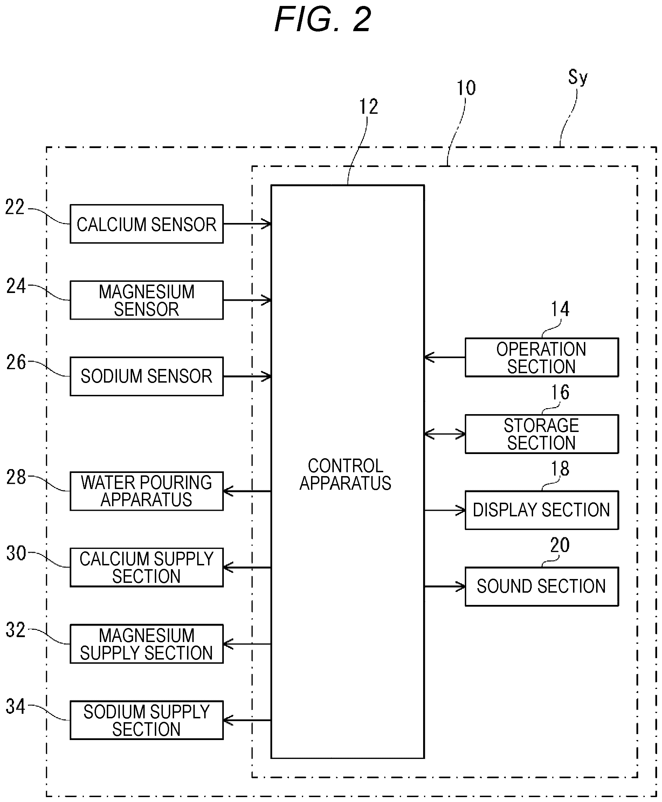

The aquaculture system Sy has an electrical configuration as illustrated in FIG. 2. Specifically, the aquaculture system Sy includes the information processing apparatus 10, various sensors (a calcium sensor 22, a magnesium sensor 24, a sodium sensor 26, etc.), various apparatuses (a water pouring apparatus 28, a calcium supply section 30, a magnesium supply section 32, and a sodium supply section 34), etc.

The information processing apparatus 10 is configured, for example, in the form of a computer system and mainly includes a control apparatus 12, an operation section 14, a storage section 16, a display section 18, a sound section 20, etc. The information processing apparatus 10 may be a portable equipment (e.g., a smartphone, a tablet terminal, or a laptop computer) or a stationary equipment (e.g., a server, a desktop personal computer, or a towertype personal computer). The control apparatus 12 includes either or both of a generalpurpose processor (e.g., a CPU) and an integrated circuit dedicated for a specific application (e.g., ASIC) and can perform various types of computations, controls, and information processing. The operation section 14 is a well-known input device such as a keyboard, a mouse, a touch panel, etc. The storage section 16 is a well-known storage device such as a ROM, a RAM, a non-volatile memory, an HDD (hard disk drive), an SSD (solid state drive), a cache memory, a work memory, etc. The display section 18 is a well-known display device such as a liquid crystal display, an organic electroluminescence display, a display lamp, etc. The sound section 20 is a device for producing sounds such as a speaker or a buzzer. Notably, although not illustrated, a communication section may be provided in the information processing apparatus 10. For example, the information processing apparatus 10 may be configured in such a manner that the control apparatus 12 and the communication section cooperate with each other so as to perform wired communication or wireless communication with an unillustrated external apparatus in accordance with a known scheme. Also, the information processing apparatus 10 is not required to be a single apparatus and a portion of the information processing apparatus 10 may be configured as a separate apparatus. For example, the control apparatus 12 and the storage section 16 may be housed in an external apparatus, and pieces of information and instructions may be exchanged between the external apparatus and the various sensors and supply sections and an interface through wired communication or wireless communication performed in accordance with a known scheme. Alternatively, the operation section 14, the display section 18, and the sound section 20 may be housed in a portable terminal which is separated from the control apparatus 12 and pieces of information and instructions may be exchanged between the control apparatus 12 and the portable terminal through wired communication or wireless communication performed in accordance with a known scheme.

Also, although not illustrated, the aquaculture system Sy includes various sensors for detecting physical parameters or chemical parameters of the rearing water in addition to the above-described mineral sensors. For example, the aquaculture system Sy includes a temperature sensor, a dissolved oxygen sensor, a pH sensor, a salt content sensor, an ammonia sensor, a nitrous acid sensor, a nitric acid sensor, a carbon dioxide sensor, a redox potential sensor, an electrical conductivity sensor, a water level sensor, etc.

### 2. Configuration for detection and adjustment of minerals

### (Basic configuration for detection and adjustment)

As shown in FIG. 2, the aquaculture system Sy includes the calcium sensor 22, the magnesium sensor 24, the sodium sensor 26, etc., as example mineral sensors.

The calcium sensor 22 is a sensor for detecting the calcium ion concentration of the rearing water W within the rearing tank 50. The calcium sensor 22 is composed of a well-known calcium ion sensor which detects the calcium ion concentration of a detection target liquid by a well-known method.

The magnesium sensor 24 is a sensor for detecting the magnesium ion concentration of the rearing water W within the rearing tank 50. The magnesium sensor 24 is composed of a well-known magnesium ion sensor which detects the magnesium ion concentration of a detection target liquid by a well-known method.

The sodium sensor 26 is a sensor for detecting the sodium ion concentration of the rearing water W within the rearing tank 50. The sodium sensor 26 is composed of a well-known sodium ion sensor which detects the sodium ion concentration of a detection target liquid by a well-known method. Notably, one or more of the calcium sensor 22, the magnesium sensor 24, the sodium sensor 26 (for example, all the sensors) may be a liquid-membrane-type mineral sensor configured in the form of a liquid-membrane-type ion sensor. When such a method is employed, a target component can be measured easily as compared with other types of sensors (for example, a sensor which employs a colorimetric analysis method using a color sensor).

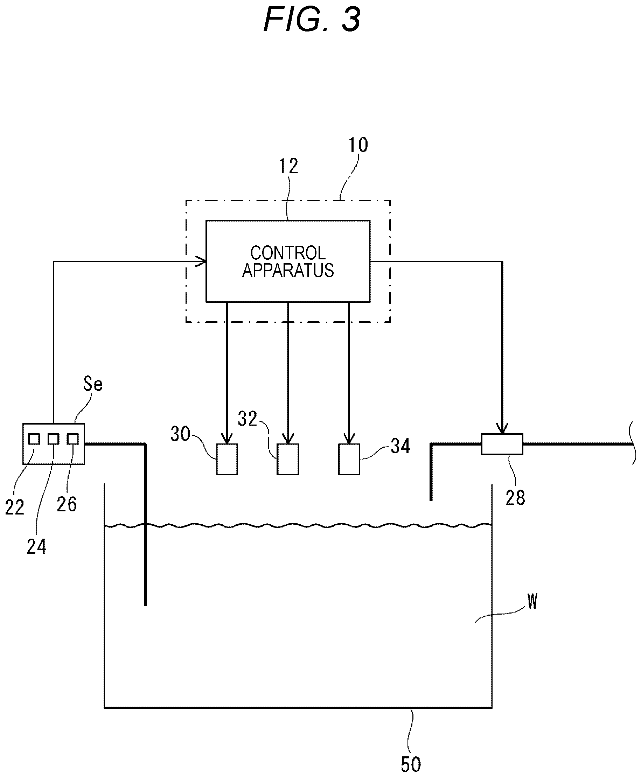

Specifically, as shown in FIG. 3, a sensor section Se configured in the form of a sensor unit is provided in the aquaculture system Sy, and the rearing water W within the rearing tank 50 is fed into the sensor section Se by an unillustrated pump. The calcium sensor 22, the magnesium sensor 24, and the sodium sensor 26 are provided in the sensor section Se and are configured to detect the concentrations of calcium, magnesium, and sodium contained in the rearing water W fed into the sensor section Se. Notably, the configuration shown in FIG. 3 is a mere example, and no limitation is imposed on the configuration so long as the calcium sensor 22, the magnesium sensor 24, and the sodium sensor 26 are arranged and configured such that these sensors can detect the concentrations of calcium, magnesium, and sodium contained in the rearing water W. For example, each sensor may be provided in the rearing tank 50 so as to detect the concentration of its target component contained in the rearing water W.

As shown in FIG. 3, the water pouring apparatus 28 is provided in the aquaculture system Sy. The water pouring apparatus 28 is an apparatus which supplies water into the rearing tank 50 and whose water pouring amount and water pouring time are controlled by the control apparatus 12.

As shown in FIG. 3, the calcium supply section 30, the magnesium supply section 32, and the sodium supply section 34 are provided in the aquaculture system Sy. The calcium supply section 30 is an apparatus for supplying calcium into the rearing tank 50 and supplies calcium into the rearing tank 50 in response to an instruction from the control apparatus 12. The supply of calcium is performed by, for example, supplying an aqueous solution which contains calcium ion in high concentration. The magnesium supply section 32 is an apparatus for supplying magnesium into the rearing tank 50 and supplies magnesium into the rearing tank 50 in response to an instruction from the control apparatus 12. The supply of magnesium is performed by, for example, supplying an aqueous solution which contains magnesium ion in high concentration. The sodium supply section 34 is an apparatus for supplying sodium into the rearing tank 50 and supplies sodium into the rearing tank 50 in response to an instruction from the control apparatus 12. The supply of sodium is performed by, for example, supplying an aqueous solution which contains sodium ion in high concentration.

### (Mineral adjustment control)

Next, mineral adjustment control performed by the control apparatus 12 will be described.

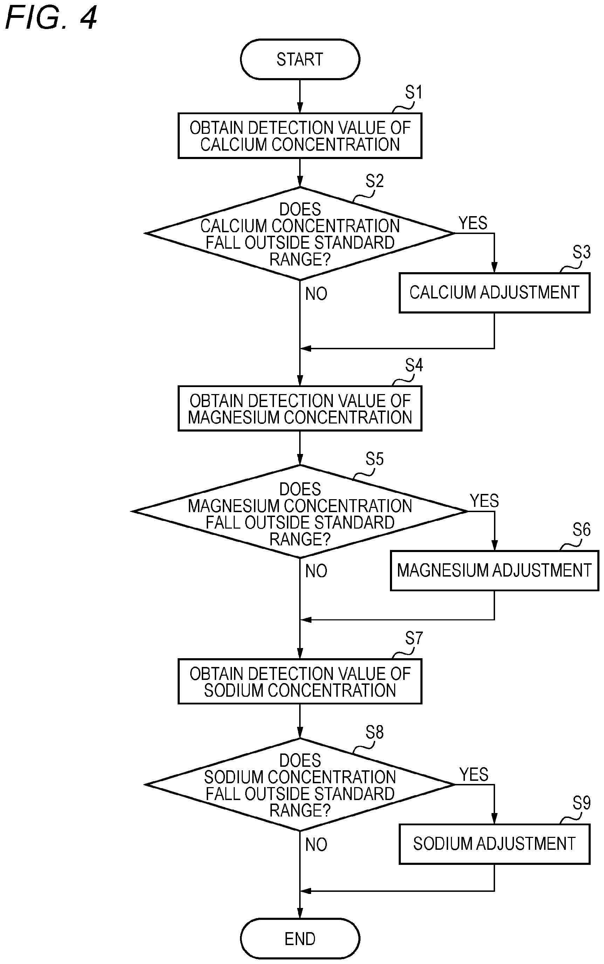

In response to satisfaction of a start condition, the control apparatus 12 reads out a control program stored in the storage section 16 and starts the mineral adjustment control shown in FIG. 4 in accordance with the control program. Notably, the start condition may be a temporal condition (for example, a condition that a predetermined time has come or a condition that a predetermined time has elapsed after the previous mineral adjustment control) or any of other conditions (for example, a condition that a predetermined operation is performed by an operator, a condition that a predetermined apparatus operates, or a condition that a predetermined step is performed). Also, the repetition interval of the control of FIG. 4 may be constant throughout the entire culturing period or may be changed in accordance with phase or the number of reared aquatic organisms.

When the control apparatus 12 has started the mineral adjustment control shown in FIG. 4, in step S1, the control apparatus 12 first obtains a calcium concentration detection value (specifically, for example, the value of the calcium ion concentration of the rearing water W detected by the calcium sensor 22 at the time point of step S1). Subsequently, after step S1, the control apparatus 12 determines whether or not the detection value obtained from the calcium sensor 22 falls outside a predetermined standard range (step S2). In the control apparatus 12, the standard range for calcium concentration is determined beforehand, for example, within a range of A1 (ppm) to A2 (ppm). In the case where the control apparatus 12 determines that the value of the calcium ion concentration of the rearing water W detected by the calcium sensor 22 at the time point of step S1 falls outside the standard range (the range of A1 (ppm) to A2 (ppm)), in step S3, the control apparatus 12 performs a process for adjusting the calcium concentration.

In the case where the control apparatus 12 performs the process of step S3, the control apparatus 12 first performs an operation for issuing a warning to the outside. The operation for issuing a warning to the outside may be performed by displaying on the display section 18 a message stating that "Calcium concentration falls outside the standard range" (a message indicating that the calcium concentration deviates from the standard range) or by producing a voice message to that effect by the sound section 20. Alternatively, the control apparatus 12 may produce an alert sound or turn on a display lamp. The alert sound and the display lamp are related to an anomalous state (calcium anomaly) in which the calcium concentration deviates from the standard range.

Furthermore, in step S3, the control apparatus 12 performs an operation for causing the calcium concentration of the rearing water to fall inside the standard range. Specifically, in the case where the calcium ion concentration Ax detected by the calcium sensor 22 is lower than the lower limit A1 of the standard range, the control apparatus 12 performs an operation for adding calcium into the rearing tank 50 in cooperation with the calcium supply section 30. For example, in the case where the detected concentration Ax is lower than the lower limit A1, the control apparatus 12 determines a calcium supply amount by using a computation table or a computation expression which determines the calcium supply amount such that the larger the difference (A1 - Ax) between the lower limit A1 and the detected concentration Ax, the larger the calcium supply amount. Subsequently, the control apparatus 12 and the calcium supply section 30 supply calcium in the state of aqueous solution or powder to the rearing tank 50 so that the amount of calcium supplied becomes equal to the determined supply amount. Notably, the above-described computation expression or the above-described computation table may be a computation expression or a computation table which determines the calcium supply amount in proportion to the above-described difference (A1 - Ax) or a computation expression or a computation table in which the calcium supply amount is determined by another calculation expression that uses the above-described difference (A1 - Ax) as a parameter.

Meanwhile, in the case where the calcium ion concentration Ax detected by the calcium sensor 22 is higher than the upper limit A2 of the standard range, the control apparatus 12 performs an operation for adding water into the rearing tank 50 in cooperation with the water pouring apparatus 28. For example, in the case where the detected concentration Ax is higher than the upper limit A2, the control apparatus 12 determines a water supply amount by using a computation table or a computation expression which determines the water supply amount such that the larger the difference (Ax - A2) between the upper limit A2 and the detected concentration Ax, the larger the water supply amount. Subsequently, the control apparatus 12 and the water pouring apparatus 28 supply water into the rearing tank 50 so that the amount of water supplied becomes equal to the determined supply amount. Notably, the above-described computation expression or the above-described computation table may be a computation expression or a computation table which determines the water supply amount in proportion to the above-described difference (Ax - A2) or a computation expression or a computation table in which the water supply amount is determined by another calculation expression that uses the above-described difference (Ax - A2) as a parameter.

Notably, in water which is supplied in the process for adjusting various ions, the concentration of the ion (in this case calcium ion) having deviated from the standard (singlepoint numerical value (standard value) or a numerical range (standard range) having a width) is 0 or lower than that of the water within the rearing tank 50. As to other components, the water which is supplied to the rearing tank 50 may or may not contain the components, or the concentrations of the components may be adjusted in accordance with the concentrations within the rearing tank 50.

When the result of the determination in step S2 is "No" or when the process of step S3 is completed, in step S4, the control apparatus 12 obtains a magnesium concentration detection value (specifically, for example, the value of the magnesium ion concentration of the rearing water W detected by the magnesium sensor 24 at the time point of step S4). Subsequently, after step S4, the control apparatus 12 determines whether or not the detection value obtained from the magnesium sensor 24 falls outside a predetermined standard range (step S5). In the control apparatus 12, the standard range for magnesium concentration is determined beforehand, for example, within a range of B1 (ppm) to B2 (ppm). In the case where the control apparatus 12 determines that the value of the magnesium ion concentration of the rearing water W detected by the magnesium sensor 24 at the time point of step S4 falls outside the standard range (the range of B1 (ppm) to B2 (ppm)), in step S6, the control apparatus 12 performs a process for adjusting the magnesium concentration.

In the case where the control apparatus 12 performs the process of step S6, the control apparatus 12 first performs the operation for issuing a warning to the outside. The operation for issuing a warning to the outside may be performed by displaying on the display section 18 a message stating that "Magnesium concentration falls outside the standard range" (a message indicating that the magnesium concentration deviates from the standard range) or by producing a voice message to that effect by the sound section 20. Alternatively, the control apparatus 12 may produce an alert sound or turn on a display lamp. The alert sound and the display lamp are related to an anomalous state (magnesium anomaly) in which the magnesium concentration deviates from the standard range.

Furthermore, in step S6, the control apparatus 12 performs an operation for causing the magnesium concentration of the rearing water to fall inside the standard range. Specifically, in the case where the magnesium ion concentration Bx detected by the magnesium sensor 24 is lower than the lower limit B1 of the standard range, the control apparatus 12 performs an operation for adding magnesium into the rearing tank 50 in cooperation with the magnesium supply section 32. For example, in the case where the detected concentration Bx is lower than the lower limit B1, the control apparatus 12 determines a magnesium supply amount by using a computation table or a computation expression which determines the magnesium supply amount such that the larger the difference (B1 - Bx) between the lower limit B1 and the detected concentration Bx, the larger the magnesium supply amount. Subsequently, the control apparatus 12 and the magnesium supply section 32 supply magnesium in the state of aqueous solution or powder into the rearing tank 50 so that the amount of magnesium supplied becomes equal to the determined supply amount. Notably, the above-described computation expression or the above-described computation table may be a computation expression or a computation table which determines the magnesium supply amount in proportion to the above-described difference (B 1 - Bx) or a computation expression or a computation table in which the magnesium supply amount is determined by another calculation expression that uses the above-described difference (B 1 - Bx) as a parameter.

Meanwhile, in the case where the magnesium ion concentration Bx detected by the magnesium sensor 24 is higher than the upper limit B2 of the standard range, the control apparatus 12 performs an operation for adding water into the rearing tank 50 in cooperation with the water pouring apparatus 28. For example, in the case where the detected concentration Bx is higher than the upper limit B2, the control apparatus 12 determines a water supply amount by using a computation table or a computation expression which determines the water supply amount such that the larger the difference (Bx - B2) between the upper limit B2 and the detected concentration Bx, the larger the water supply amount. Subsequently, the control apparatus 12 and the water pouring apparatus 28 supply water into the rearing tank 50 so that the amount of water supplied becomes equal to the determined supply amount. Notably, the above-described computation expression or the above-described computation table may be a computation expression or a computation table which determines the water supply amount in proportion to the above-described difference (Bx - B2) or a computation expression or a computation table in which the water supply amount is determined by another calculation expression that uses the above-described difference (Bx - B2) as a parameter.

When the result of the determination in step S5 is "No" or when the process of step S6 is completed, in step S7, the control apparatus 12 obtains a sodium concentration detection value (specifically, for example, the value of the sodium ion concentration of the rearing water W detected by the sodium sensor 26 at the time point of step S7). Subsequently, after step S7, the control apparatus 12 determines whether or not the detection value obtained from the sodium sensor 26 falls outside a predetermined standard range (step S8). In the control apparatus 12, the standard range for sodium concentration is determined beforehand, for example, within a range of C1 (ppm) to C2 (ppm). In the case where the control apparatus 12 determines that the value of the sodium ion concentration of the rearing water W detected by the sodium sensor 26 at the time point of step S7 falls outside the standard range (the range of C1 (ppm) to C2 (ppm)), in step S9, the control apparatus 12 performs a process for adjusting the sodium concentration.

In the case where the control apparatus 12 performs the process of step S9, the control apparatus 12 first performs the operation for issuing a warning to the outside. The operation for issuing a warning to the outside may be performed by displaying on the display section 18 a message stating that "Sodium concentration falls outside the standard range" (a message indicating that the sodium concentration deviates from the standard range) or by producing a voice message to that effect by the sound section 20. Alternatively, the control apparatus 12 may produce an alert sound or turn on a display lamp. The alert sound and the display lamp are related to an anomalous state (sodium anomaly) in which the sodium concentration deviates from the standard range.

Furthermore, in step S9, the control apparatus 12 performs an operation for causing the sodium concentration of the rearing water to fall inside the standard range. Specifically, in the case where the sodium ion concentration Cx detected by the sodium sensor 26 is lower than the lower limit C1 of the standard range, the control apparatus 12 performs an operation for adding sodium into the rearing tank 50 in cooperation with the sodium supply section 34. For example, in the case where the detected concentration Cx is lower than the lower limit C1, the control apparatus 12 determines a sodium supply amount by using a computation table or a computation expression which determines the sodium supply amount such that the larger the difference (C1 - Cx) between the lower limit C1 and the detected concentration Cx, the larger the sodium supply amount. Subsequently, the control apparatus 12 and the sodium supply section 34 supply sodium in the state of aqueous solution or powder into the rearing tank 50 so that the amount of sodium supplied becomes equal to the determined supply amount. Notably, the above-described computation expression or the above-described computation table may be a computation expression or a computation table which determines the sodium supply amount in proportion to the above-described difference (C1 - Cx) or a computation expression or a computation table in which the sodium supply amount is determined by another calculation expression that uses the above-described difference (C1 - Cx) as a parameter.

Meanwhile, in the case where the sodium ion concentration Cx detected by the sodium sensor 26 is higher than the upper limit C2 of the standard range, the control apparatus 12 performs an operation for adding water into the rearing tank 50 in cooperation with the water pouring apparatus 28. For example, in the case where the detected concentration Cx is higher than the upper limit C2, the control apparatus 12 determines a water supply amount by using a computation table or a computation expression which determines the water supply amount such that the larger the difference (Cx - C2) between the upper limit C2 and the detected concentration Cx, the larger the water supply amount. Subsequently, the control apparatus 12 and the water pouring apparatus 28 supply water into the rearing tank 50 so that the amount of water supplied becomes equal to the determined supply amount. Notably, the above-described computation expression or the above-described computation table may be a computation expression or a computation table which determines the water supply amount in proportion to the above-described difference (Cx - C2) or a computation expression or a computation table in which the water supply amount is determined by another calculation expression that uses the above-described difference (Cx - C2) as a parameter.

In the present configuration, the control apparatus 12 corresponds to an example of the adjustment section and functions in such a manner that, when the concentration of a mineral detected by a corresponding mineral sensor has deviated from its standard range, the control apparatus 12 performs instruction issuance or operation for causing the concentration of the mineral contained in the rearing water to fall inside its standard range.

### 3. Effects of the present configuration

In the above-described aquaculture system Sy, the concentration of a mineral contained in the rearing water W can be detected by a corresponding mineral sensor, and, when the concentration of the mineral detected by the mineral sensor deviates from its standard range, instruction issuance or operation for causing the mineral concentration to fall inside its standard range can be performed by the control apparatus 12 (the adjustment section). As a result, it is possible to optimize the mineral concentration of the rearing water W, thereby preventing the aquaculture system Sy from being operated in a state in which the mineral concentration falls outside the standard range.

Also, in the aquaculture system Sy, in the case where the mineral concentration of the rearing water W is lower than the lower limit of the standard range; namely, in the case where the mineral is insufficient, instruction issuance or operation for making up for the shortage can be performed by the control apparatus 12 (the adjustment section). As a result, it is possible to prevent the aquaculture system Sy from being operated continuously in a state in which the mineral concentration of the rearing water W is excessively low, thereby restraining occurrence of a problem due to excessive continuation of a period during which the mineral concentration of the rearing water W is excessively low.

Also, in the aquaculture system Sy, in the case where the mineral concentration of the rearing water W is higher than the upper limit of the standard range; namely, in the case where the mineral is excessive, instruction issuance or operation for remedying the mineral excessive state can be performed by the control apparatus 12 (the adjustment section). As a result, it is possible to prevent the aquaculture system Sy from being operated continuously in a state in which the mineral concentration of the rearing water W is excessively high, thereby restraining occurrence of a malfunction due to excessive continuation of a period during which the mineral concentration of the rearing water W is excessively high.

Also, in the aquaculture system Sy, in the case where the mineral concentration of the rearing water W deviates from the standard range, instruction issuance or operation for issuing a warning to the outside can be performed by the control apparatus 12 (the adjustment section). Therefore, an operator or the like who has recognized the warning operation can grasp the anomaly of the mineral concentration. Accordingly, the operator can quickly take a countermeasure suitable for the case where the mineral concentration of the rearing water W deviates from the standard range.

Also, in the aquaculture system Sy, the aquatic organism to be reared is a crustacean. In the case where the crustacean is reared in the rearing water W, the concentration of the mineral contained in the rearing water W is more important, and when the period during which the mineral concentration is not proper becomes excessively long, the risk that the crustacean is not reared properly becomes higher. Specifically, for example, the problem that the crustacean fails to shed its old shell at a proper speed and the problem that the crustacean is not grown to a proper size at a proper timing become more likely to occur. In this regard, since the aquaculture system Sy can optimize the mineral concentration of the rearing water W and it is possible to prevent the aquaculture system Sy from being operated continuously in a state in which the mineral concentration falls outside the standard range, it is possible to reduce the risk that the crustacean is not grown properly and to stabilize and accelerate the growth of the crustacean.

### <Second embodiment>

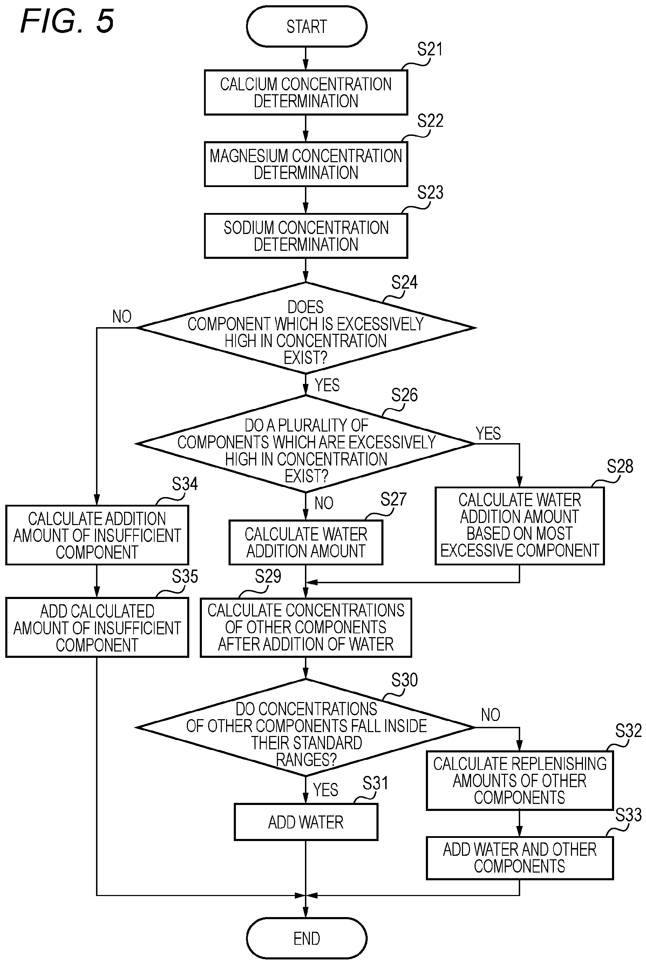

An aquaculture system Sy according to a second embodiment is identical with the aquaculture system Sy of the first embodiment except for the point that the mineral adjustment control shown in FIG. 4 is changed as shown in FIG. 5. For example, the aquaculture system Sy according to the second embodiment is identical with the aquaculture system Sy of the first embodiment in terms of the matters described in the above-described section entitled "1. Outline of aquaculture system." Moreover, the aquaculture system Sy according to the second embodiment is identical with the aquaculture system Sy of the first embodiment in terms of the matters described in a subsection entitled "(Basic configuration for detection and adjustment)" in the above-described section entitled "2. Configuration for detection and adjustment of minerals." Also, as to the configurations shown in FIGS. 1 to 3, the aquaculture system Sy according to the second embodiment is identical with the aquaculture system Sy of the first embodiment. Therefore, in the following description, FIGS. 1 to 3 will be referred to when necessary.

In the aquaculture system Sy (FIG. 1) according to the second embodiment, the control apparatus 12 (FIG. 1) performs the mineral adjustment control in accordance with the flow shown in FIG. 5.

The control apparatus 12 shown in FIG. 1 starts the mineral adjustment control of FIG. 5 in response to satisfaction of a start condition. Notably, the start condition may be a temporal condition (for example, a condition that a predetermined time has come or a condition that a predetermined time has elapsed after the previous mineral adjustment control) or any of other conditions (for example, a condition that a predetermined operation is performed by an operator, a condition that a predetermined apparatus operates, or a condition that a predetermined step is performed). Also, the repetition interval of the control of FIG. 5 may be constant throughout the entire culturing period or may be changed in accordance with phase or the number of reared aquatic organisms.

When the control apparatus 12 has started the mineral adjustment control shown in FIG. 5, in step S21, the control apparatus 12 first performs determination as to calcium concentration. The process of step S21 is identical with, for example, the processes of steps S1 and S2 in FIG. 4. Specifically, the control apparatus 12 obtains the value of the calcium ion concentration of the rearing water W detected by the calcium sensor 22 at the time point of step S21 and determines whether or not the value of the calcium ion concentration (the detection value obtained from the calcium sensor 22) falls outside a predetermined calcium ion concentration standard range (the range of A1 (ppm) to A2 (ppm)).

After step S21, the control apparatus 12 performs determination as to magnesium concentration in step S22. The process of step S22 is identical with, for example, the processes of steps S4 and S5 in FIG. 4. Specifically, the control apparatus 12 obtains the value of the magnesium ion concentration of the rearing water W detected by the magnesium sensor 24 at the time point of step S22 and determines whether or not the value of the magnesium ion concentration (the detection value obtained from the magnesium sensor 24) falls outside a predetermined magnesium ion concentration standard range (the range of B1 (ppm) to B2 (ppm)).

After step S22, the control apparatus 12 performs determination as to sodium concentration in step S23. The process of step S23 is identical with, for example, the processes of steps S7 and S8 in FIG. 4. Specifically, the control apparatus 12 obtains the value of the sodium ion concentration of the rearing water W detected by the sodium sensor 26 at the time point of step S23 and determines whether or not the value of the sodium ion concentration (the detection value obtained from the sodium sensor 26) falls outside a predetermined sodium ion concentration standard range (the range of C1 (ppm) to C2 (ppm)).

After step S23, the control apparatus 12 determines in step S24 whether or not a component whose concentration is higher than its standard range (a component whose concentration is excessively high) has been detected in the determinations in steps S21 to S23. In the case where the control apparatus 12 determines in step S21 that the calcium ion concentration is equal to or lower than the upper limit (A2 (ppm)) of the standard range (the range of A1 (ppm) to A2 (ppm)), determines in step S22 that the magnesium ion concentration is equal to or lower than the upper limit (B2 (ppm)) of the standard range (the range of B1 (ppm) to B2 (ppm)), and determines in step S23 that the sodium ion concentration is equal to or lower than the upper limit (C2 (ppm)) of the standard range (the range of C1 (ppm) to C2 (ppm)), the control apparatus 12 makes a "No" determination in step S24 and performs the process of step S34.

In step S34, the control apparatus 12 calculates an addition amount (supply amount) of each insufficient component. Subsequently, in step S35, the control apparatus 12 performs instruction issuance or operation for adding the insufficient component in the addition amount (supply amount) determined in step S34. In the case where, in the determinations of steps S21, S22, and S23, all the components are determined to be equal to or lower than the upper limits determined for the components and all the components are determined to be equal to or higher than the lower limits determined for the components; i.e., all the components fall inside their standard ranges, in step S34, the control apparatus 12 sets the addition amount (supply amount) of each insufficient component to 0. In this case, in step S35, the control apparatus 12 does not perform the instruction issuance or operation for adding an insufficient component(s). In this case, in step S35, the control apparatus 12 may report that all the components satisfy their standard ranges. For example, the control apparatus 12 may display on the display section 18 a message stating that "Concentrations of all the components are proper" or provide a voice message to that effect by the sound section 20.

In the case where the control apparatus 12 executes the process of step S34 after having determined in steps S21, S22, and S23 that one of the components is lower than the lower limit determined for that component, in step S34, the control apparatus 12 calculates the addition amount (supply amount) of that component (insufficient component). The addition amount (supply amount) in this case is an amount determined such that, when the insufficient component is added in that addition amount to the rearing water W within the rearing tank 50, the concentration of the insufficient component falls inside the standard range. Specifically, the addition amount (supply amount) may be determined such that, when the insufficient component is added in that addition amount to the rearing water W, the concentration of the insufficient component becomes equal to a predetermined target value within the standard range. The target value in this case may be, for example, the center value of the standard range or another value (the upper limit or the lower limit). Specifically, on the basis of the concentration of an insufficient component whose ion concentration is lower than the standard range in the rearing water W at the present (before addition of the insufficient component) (i.e., at the time of start of step S34), the amount Zw of the rearing water W at the present (before addition of the insufficient component), and a target value of the ion concentration of the above-described insufficient component, the control apparatus 12 calculates the addition amount of the insufficient component such that the ion concentration of the insufficient component becomes equal to the above-described target value (the target value of the ion concentration of the insufficient component) after addition of the insufficient component. For example, in the case where the control apparatus 12 performs the process of step S34 when the detected calcium ion concentration Ax is determined in step S21 to be lower than the lower limit of the calcium ion concentration, since calcium is the insufficient component, in step S34, the control apparatus 12 calculates an addition amount (supply amount) Xc of calcium, which is the insufficient component. This addition amount Xc is an amount determined such that, when calcium is added in the addition amount Xc, the calcium ion concentration of the calcium-added rearing water becomes equal to the above-described target value At. Therefore, when the calcium ion concentration Ax before addition of calcium, the amount Zw of the rearing water W within the rearing tank 50, and the target value At are determined, the addition amount Xc is determined univocally. Accordingly, the control apparatus 12 determines the addition amount Xc of calcium such that the calcium ion concentration becomes equal to the above-described target value At after addition of calcium, on the basis of the calcium ion concentration Ax in the rearing water W at the present (before addition of calcium), the amount Zw of the rearing water W within the rearing tank 50 at the present (before addition of calcium), and the target value At of the calcium ion concentration.

After having calculated the addition amount (supply amount) of the insufficient component in step S34, in step S35, the control apparatus 12 performs instruction issuance or operation for adding a mineral to the rearing water W as the insufficient component. The addition of the mineral in this case can be performed in the same manner as in the first embodiment. For example, in the case where calcium ion is the insufficient component and the addition amount (supply amount) Xc is determined in the above-described manner in step S34, in step S35, the control apparatus 12 and the calcium supply section 30 supply calcium in the state of aqueous solution or powder into the rearing tank 50 so that the amount of calcium supplied becomes equal to the determined addition amount Xc. Alternatively, instead of such an operation, in step S35, the control apparatus 12 may cooperate with the display section 18 or the sound section 20 so as to display on the display section 18 a message stating that "Please supply calcium of Xc (g)" or the like or to provide a voice message to that effect by the sound section 20.

Notably, in the case where two or more kinds of components are determined to be insufficient components in step S34, the control apparatus 12 calculates respective addition amounts (respective supply amounts) of the two or more kinds of components in step S34 by the same method as the above-described method. Subsequently, in step S35, the control apparatus 12 performs instruction issuance or operation for adding minerals corresponding to the two or more kinds of insufficient components on the basis of the respective addition amounts (respective supply amounts) of the insufficient components calculated in step S34. In this example, in step S35, the control apparatus 12 can perform instruction issuance or operation for causing the concentrations of the plurality of types of minerals to fall inside their standards. Also, in the case where the control apparatus 12 performs instruction issuance or operation for adding two or more kinds of minerals to the rearing water W, the control apparatus 12 may calculate respective addition amounts (respective supply amounts) of the two or more kinds of to-be-added minerals in step S34 as described above and then in step S35 perform instruction issuance or operation for adding to the rearing water W the two or more types of minerals mixed together on the basis of the respective addition amounts (respective supply amounts) calculated in step S34. For example, in the case where calcium ion and magnesium ion are determined to be insufficient components in step S34, the control apparatus 12 may calculate the addition amount (supply amount) Xc of calcium (insufficient component) and the addition amount (supply amount) Xm of magnesium (insufficient component) in step S34 by the above-described method, and perform control (operation) for activating the calcium supply section 30 and the magnesium supply section 32 in subsequent step S35 so as to add calcium and magnesium to the rearing water W after mixing calcium and magnesium at a mixing ratio (mixing ratio between the calcium addition amount Xc and the magnesium addition amount Xm) determined on the basis of the respective addition amounts (respective supply amounts) Xc and Xm calculated in step S34. In this example, the control apparatus 12, the calcium supply section 30, and the magnesium supply section 32 operate in such a manner as to add all the types of to-be-added minerals (calcium and magnesium) to the rearing water W after mixing them at the mixing ratio (mixing ratio between the calcium addition amount Xc and the magnesium addition amount Xm) determined on the basis of the respective addition amounts (respective supply amounts) Xc and Xm calculated in step S34. Notably, no limitation is imposed on the "operation for adding all the types of to-be-added minerals to the rearing water W after mixing" so long as respective periods during which the plurality of minerals to be mixed are supplied, respectively, at least partially overlap each other. The plurality of minerals may be supplied from the same supply passage or supplied from different supply passages. Notably, in the present specification, the control apparatus 12 corresponds to an example of the "adjustment section." However, the "adjustment section" may include supply sections for supplying the minerals (for example, the calcium supply section 30, the magnesium supply section 32, and the sodium supply section 34). Alternatively, instead of such an operation, in step S35, the control apparatus 12 may provide a notice from which a user can recognize or specify the respective addition amounts (respective supply amounts) calculated in step S34. For example, in step S35, the control apparatus 12 may display on the display section 18 a message stating that "Please mix and add calcium Xc (g) and magnesium Xm (g)" or the like, or provide a voice message to that effect by the sound section 20. Notably, in the case where the operation for adding the minerals to the rearing water W is performed in step S35, etc., the operation for adding the minerals may be performed at one time or may be dividedly performed in a plurality of times.

In the case where the control apparatus 12 determines in step S21 that the calcium ion concentration is higher than its standard range (the range of A1 (ppm) to A2 (ppm), determines in step S22 that the magnesium ion concentration is higher than its standard range (the range of B1 (ppm) to B2 (ppm), or determines in step S23 that the sodium ion concentration is higher than its standard range (the range of C1 (ppm) to C2 (ppm), the control apparatus 12 makes a "Yes" determination in step S24 and performs the process of step S26.

In step S26, the control apparatus 12 determines whether or not a plurality of components whose concentrations are higher than their standard ranges (excessive components) have been detected in the determinations of steps S21 to S23. In the case where the control apparatus 12 determines in the process of step S26 that only one component whose concentration is higher than its standard range has been detected in the determinations of steps S21 to S23, the control apparatus 12 makes a "No" determination in step S26 and performs the process of step S27.

In step S27, the control apparatus 12 calculates a water addition amount Xw. The water addition amount Xw in this case is an amount determined in such a manner that, when water is added to the rearing water W within the rearing tank 50 such that the amount Zw of the rearing water W at the present (before addition of water) increases by the water addition amount Xw, the concentration of the excessive component falls inside a standard range determined for that excessive component. Specifically, the water addition amount Xw may be an amount determined in such a manner that, when water is added in the above-described water addition amount Xw to the rearing water W whose current amount is Zw, the concentration of the excessive component coincides with the above-described target value within the standard range of the excessive component. Accordingly, on the basis of the concentration of a component of interest (excessive component) which is excessive in the rearing water W at the present (before addition of water) (i.e., at the point in time when the execution of step S27 is started), the amount Zw of the rearing water W at the present (before addition of water), and the target value of the concentration of the component of interest, the control apparatus 12 calculates the water addition amount Xw such that the concentration of the excessive component of interest becomes equal to the above-described target value after addition of water. For example, in the case where the control apparatus 12 performs the process of step S27 when the detected calcium ion concentration Ax is determined in step S21 to be higher than the upper limit (A2 (ppm)) of the standard range (the range of A1 (ppm) to A2 (ppm)), in step S27, the control apparatus 12 calculates the above-described water addition amount Xw such that, when water of the water addition amount Xw is added, the calcium ion concentration of the water-added rearing water becomes equal to the above-described target value At. When the calcium ion concentration Ax at the present (before addition of water) (i.e., at the point in time when the execution of step S27 is started), the amount Zw of the rearing water W within the rearing tank 50 at the present (before addition of water), and the target value At are determined, the water addition amount Zw is determined univocally. Accordingly, the control apparatus 12 determines the water addition amount Xw such that the calcium ion concentration becomes equal to the target value At after addition of water, on the basis of the calcium ion concentration Ax in the rearing water W at the present (before addition of water), the amount Zw of the rearing water W within the rearing tank 50 at the present (before addition of water), and the target value At. Notably, the amount Zw of the rearing water W within the rearing tank 50 can be detected by a well-known method. For example, in the case where the inner edge shape of the rearing tank 50 is constant, if the level of the rearing water W within the rearing tank 50 can be detected by a water level sensor, the amount Zw of the rearing water W can be determined. The method for determining the amount Zw of the rearing water W is not limited to this method. For example, the amount Zw of the rearing water W at the present may be determined by detecting the amount of water flowing into the rearing tank 50 and the amount of water flowing out from the rearing tank 50 by using water meters.

In the case where the control apparatus 12 determines in the process of step S26 that a plurality of components whose concentrations are higher than their standard ranges have been detected in the determinations of steps S21 to S23, the control apparatus 12 makes a "Yes" determination in step S26 and performs the process of step S28.

In step S28, the control apparatus 12 calculates the water addition amount Xw on the basis of a component whose ion concentration is most excessive at the time point of start of step S28 (hereinafter referred to as the "most excessive component"). The "most excessive component" is a component which is one of a plurality of components whose ion concentration are excessive at the time point of start of step S28 and which requires the largest amount of water for remedying the state in which the ion concentration is excessively high and for bringing the excessively high ion concentration back to the target value. In step S28, first, on the basis of the concentration of the component of interest (excessive component) which is excessive in the rearing water W at the present (before addition of water) (i.e., at the time of start of step S28), the amount Zw of the rearing water W at the present (before addition of water), and the target value of the concentration of the component of interest, the control apparatus 12 calculates the amount of water which must be added so as to render the excessively high concentration of the component of interest equal to the above-described target value after addition of water (hereinafter the amount of water which must be added will be referred to as the "necessary water amount"). When attention is paid to a certain "excessive component," the necessary water amount is an amount determined such that, when water of the necessary water amount is added to the rearing water W whose current amount is Zw, the concentration of the excessive component becomes equal to the target value determined for that excessive component. For example, in the case where attention is paid to calcium ion as an excessive component, the necessary water amount is an amount determined such that, when water of the necessary water amount is added to the rearing water W whose current amount is Zw, the concentration of calcium ion becomes equal to the target value At determined for calcium ion. The control apparatus 12 computes such a necessary water amount by paying attention to each of the plurality of components of interest (excessive components) and chooses a component whose necessary water amount is the largest as the "most excessive component."

The water addition amount Xw calculated in step S28 is an amount determined such that, when water of the water addition amount Xw is added to the rearing water W in the rearing tank 50, whose current amount is Zw, the concentration of the above-described "most excessive component" becomes equal to the target value determined for the "most excessive component." The control apparatus 12 calculates the water addition amount Xw such that the concentration of the "most excessive component" becomes equal to the above-described target value after addition of water, on the basis of the concentration of the "most excessive component" in the rearing water W at the present (before addition of water) (i.e., at the time of start of step S28), the amount Zw of the rearing water W at the present (before addition of water), and the target value of the concentration of the "most excessive component." For example, in the case where the "most excessive component" is calcium ion, the control apparatus 12 calculates the water addition amount Xw such that the calcium ion concentration becomes equal to the target value At after addition of water, on the basis of the calcium ion concentration Ax of the rearing water W at the present (before addition of water) (i.e., the time of start of step S28), the amount Zw of the rearing water W within the rearing tank 50 at the present (before addition of water), and the above-described target value At.

After step S27 or step S28, the control apparatus calculates the concentrations of other components after addition of water under the assumption that water of the above-described water addition amount Xw has been added to the rearing water W within the rearing tank 50. In the case where the process of step S29 is performed after step S27, other components are components other than the component determined to be an excessive component in the step S27. In the case where the process of step S29 is performed after step S28, other components are components other than the component determined to be the "most excessive component" in the step S28. For example, in the case where the excessive component determined in step S27 or the "most excessive component" determined in the step S28 is calcium ion, the "other components" in step S29 are magnesium ion and sodium ion. In step S29, the control apparatus calculates the respective concentrations of the above-described other components for the case where water of the water addition amount Xw calculated in step S27 or S28 is added to the rearing water W whose current amount is Zw. Notably, since the current concentrations of the other components in the rearing water W within the rearing tank 50 have already been determined, when the amount ((Zw + Xw) of the rearing water within the rearing tank 50 to which water of the water addition amount Xw has been added is determined, the respective concentrations of the above-described other components after addition of water of the water addition amount Xw are determined.

After step S29, the control apparatus 12 determines in step S30 whether or not the concentrations of the other components obtained in step S29 (the concentrations of the other components after addition of water under the assumption that water of the water addition amount Xw has been added to the rearing tank 50) fall inside their standard ranges. For example, in the case where magnesium ion is one of the other components in step S29, the control apparatus 12 determines in step S30 whether or not the magnesium ion concentration obtained in step S29 (the magnesium ion concentration after addition of water under the assumption that water of the water addition amount Xw has been added to the rearing tank 50) falls inside its standard range (the range of (B1 (ppm) to B2 (ppm)).

In the case where the control apparatus 12 determines in step S30 that the concentrations of all the other components fall inside their standard ranges, in step S31, the control apparatus 12 performs instruction issuance or operation for adding water of the above-described water addition amount Xw. In the case where the control apparatus 12 performs the process of step S31, the control apparatus 12 supplies water of the water addition amount Xw to the rearing tank 50 in cooperation with the water pouring apparatus 28. Alternatively, the control apparatus 12 may cooperate with the display section 18 or the sound section 20 so as to display on the display section 18 a message stating that "Please pouring water of Xw (g)" or the like or to provide a voice message to that effect by the sound section 20.

In the case where the control apparatus 12 determines in step S30 that the concentration of at least one of the other components falls outside its standard range (lower than its lower limit), in step S32, the control apparatus 12 calculates a replenishing amount of this component (another component which becomes an insufficient component). In this case, on the basis of the concentration of an ion which becomes an insufficient component as a result of addition of water of the water addition amount Xw, the amount Zw of the rearing water W at the present (before addition of water), the water addition amount Xw, and the target value of the ion concentration of the above-described insufficient component, the control apparatus 12 calculates the addition amount of the insufficient component such that the ion concentration of the insufficient component becomes equal to the above-described target value (the target value of the ion concentration of the insufficient component) after addition of "water of the water addition amount Xw" and the "insufficient component of the addition amount." For example, in the case where calcium ion is one insufficient component of the "other components" (a component whose ion concentration becomes lower than its lower limit when water of the water addition amount Xw is added to the current rearing water W whose current amount is Zw), in step S32, the control apparatus 12 calculates the addition amount Xc of calcium such that the calcium ion concentration becomes equal to the above-described target value At after addition of "water of the water addition amount Xw" and "calcium of the addition amount Xc," on the basis of the calcium ion concentration Ax of the rearing water W at the present (before addition of water) (i.e., at the time of start of step S32), the amount Zw of the rearing water W at the present (before addition of water), the water addition amount Xw, and the target value At of the calcium ion concentration. Notably, in the case where the control apparatus 12 determines in step S30 that a plurality of components become insufficient components (components whose ion concentrations become lower than their lower limits) as a result of addition of water of the water addition amount Xw, the control apparatus 12 may calculate the above-described addition amount for each of the insufficient components.

After step S32, in step S33, the control apparatus 12 performs instruction issuance or operation for adding "water of the water addition amount Xw" and "one or more insufficient components" to the rearing water W. In step S33, the control apparatus 12 can perform operation or instruction issuance for adding water of the water addition amount Xw in the same manner as in step S31. Also, the control apparatus 12 can perform operation or instruction issuance for adding an insufficient mineral(s) in the same manner as in step S35. The operation or instruction issuance for adding "water of the water addition amount Xw" to the rearing water W and the operation or instruction issuance for adding "one or more insufficient minerals" to the rearing water W are desirably performed in the same period. By adding "water of the water addition amount Xw" and "one or more insufficient minerals" to the rearing water W in the same period, it is possible to adjust the concentrations of the minerals while preventing or mitigating deviation of the concentrations of the minerals from their standard ranges. The "same period" means that at least a portion of the period during which water is added overlaps the period during which one or more insufficient components are added.

As described above, in the case where the control apparatus 12 performs instruction issuance or operation for adding water to the rearing water W, before performing the instruction issuance or operation for adding water to the rearing water W, the control apparatus 12 calculates the necessary water addition amount Xw (water supply amount) in step S27 or S28 and calculates the "concentrations of other components" in subsequent step S29, thereby predicting changes in the concentrations of the plurality of minerals as a result of addition of water of the water addition amount Xw (water supply amount). In the case where the control apparatus 12 performs such a prediction, when it is predicted that, as a result of addition of water of the water addition amount (water supply amount) Xw, the concentration of at least one of the plurality of kinds of minerals becomes lower than its standard concentration, in step S32, the control apparatus 12 calculates the "replenishing amounts of other components" as the mineral supply amount necessary for rendering the concentration of the above-described "at least one kind of mineral" coincident with the standard concentration. Subsequently, in step S33, the control apparatus 12 performs instruction issuance or operation for adding water on the basis of the calculated water addition amount Xw (water supply amount) and performs instruction issuance or operation for adding the "at least one kind of mineral" on the basis of the mineral supply amount (the "replenishing amounts of other components") calculated in step S32. Notably, even in the case where the control apparatus 12 performs instruction issuance or operation for adding two or more kinds of minerals to the rearing water W in step S33, the control apparatus 12 can perform the instruction issuance or operation in the same manner as the instruction issuance or operation for adding two or more kinds of minerals to the rearing water in step S35. For example, the control apparatus 12 can perform instruction issuance or operation for adding to the rearing water W two or more kinds of minerals mixed together on the basis of the "supply amounts of two or more kinds of minerals calculated in step S32." Meanwhile, in the case where the control apparatus 12 performs the above-described prediction in step S29 and makes a "Yes" determination in step S30 (i.e., it is predicted that none of the plurality of kinds of minerals becomes lower in concentration than the standard, in step S31, the control apparatus 12 performs instruction issuance or operation for adding water on the basis of the calculated water addition amount Xw (water supply amount).

### <Other embodiments>

The present invention is not limited to the embodiment described by the above description with reference to the drawings. For example, the features of the above-described embodiment and embodiments which will be described below can be combined in any manner so long as no contradiction occurs. Also, any of the features of the above-described embodiment and embodiments which will be described below may be omitted unless explicitly stated as essential. Moreover, the above-described embodiment may be modified as follows.

In the above-described embodiment, the calcium sensor, the magnesium sensor, and the sodium sensor are exemplified as mineral sensors. However, only one or two of these sensors may be provided. Alternatively, other types of mineral sensors may be provided in addition to or in place of these mineral sensors.

In the above-described embodiment, shrimp is exemplified as an aquatic organism cultured in the aquaculture system Sy. However, the aquatic organism may be a crustacean other than shrimp (crab, krill, etc.), or other aquatic organisms such as fishes, shellfishes, aquatic animals, marine mammals, seaweeds, etc. Also, the culture to which the aquaculture system is applied may be "aquaculture in which an aquatic product is cultured in water containing salt contents" or "aquaculture in which an aquatic product is cultured in water containing no salt content."

In the above-described embodiment, the circulation passage 70 is exemplified as an example of the circulation passage. However, its configuration is not limited to the configuration of FIG. 1 and any of various configurations may be employed so long as the circulation passage is a passage through which the rearing water within the rearing tank circulates outside the rearing tank and returns to the rearing tank.

In the above-described embodiment, the mineral sensors detect the mineral concentrations of the rearing water within the rearing tank 50. However, the mineral sensors may detect the mineral concentrations of the rearing water within the circulation passage 70. For example, the mineral sensors may detect the mineral concentrations of the rearing water within the sedimentation tank 52 or may detect the mineral concentrations of the rearing water within the pipe lines 66A to 66G.

In the above-described embodiment, in the case where the concentration of a certain mineral is lower than the lower limit of its standard range, in step S3, S6, or S9, the mineral is supplied in a supply amount corresponding to the difference between the detected mineral concentration and the lower limit. However, in the case where mineral concentration is lower than the lower limit of its standard range, the mineral may be added in a predetermined fixed amount.

In the above-described embodiment, in the case where the concentration of a certain mineral is higher than the upper limit of its standard range, in step S3, S6, or S9, water is supplied in a supply amount corresponding to the difference between the detected mineral concentration and the upper limit. However, in the case where mineral concentration is higher than the upper limit of its standard range, water may be added in a predetermined fixed amount.

In the above-described embodiment, as an example of the operation for causing the mineral concentrations of the rearing water to fall inside their standard ranges, there is shown the operation for supplying a mineral in step S3, S6, or S9 when the concentration of the mineral is lower than its lower limit. However, instead of performing the mineral supply operation, the control apparatus may perform "instruction issuance for causing the "mineral concentrations of the rearing water to fall inside their standard ranges." For example, in the above-described embodiment, when the calcium concentration is lower than its lower limit, the control apparatus performs the calcium supply operation in step S3. However, instead of the calcium supply operation, the control apparatus may instruct an operator to supply calcium by means of display or sound. For example, the control apparatus may display a message stating that "Please supply calcium" on the display section 18 or provide a voice message to that effect by the sound section 20. Alternatively, the control apparatus may calculate the calcium supply amount in the same manner as in the above-described embodiment and display the calculated supply amount on the display section 18 by, for example, displaying a message stating that "Please supply calcium of xx g" or provide a voice message to that effect by the sound section 20. The adjustment of magnesium in step S6 and the adjustment of sodium in step S9 can be performed in the same manner. Also, in the above-described embodiment, in the case where the calcium concentration is higher than its upper limit, the control apparatus performs the water pouring operation in step S3. However, instead of the water pouring operation, the control apparatus may instruct the operator to supply water by means of display or sound. For example, the control apparatus may display a message stating that "Please pour water" on the display section 18 or provide a voice message to that effect by the sound section 20. Alternatively, the control apparatus may calculate the water pouring amount in the same manner as in the above-described embodiment and display the calculated pouring amount on the display section 18 by, for example, displaying a message stating that "Please pour water of xx g" or provide a voice message to that effect by the sound section 20. Also, at the time of the water pouring operation, discharge of water may be performed for changing the water. Also, in the above-described embodiment, the control apparatus displays a directly instructing message, such as a message stating that "Please supply XX," as an "instruction for causing a certain mineral concentration of the rearing water to fall inside its standard range." However, instead of the directly instructing message, the control apparatus may display a message for calling the operator's attention to the operation of causing the mineral concentration to fall inside its standard range; for example, a warning display stating that "XX is insufficient" or "XX is excessive" or a recommendation message stating that "Supply of XX is recommended." Also, the messages displayed on the display section 18 are not limited to sentences. The display of messages maybe performed by using images and/or animations. For example, icons showing minerals and water may be displayed in a frame labeled "Supply is required." Alternatively, an animation showing supply of an insufficient mineral component may be displayed.

In the above-described embodiment, there is shown an example in which, for adjustment of the mineral concentrations of the rearing water, a standard value (a standard range which is a numerical range serving as a standard) is determined for each mineral, and, when the concentration of a certain mineral deviates from the upper or lower limit of the standard value, concentration adjustment operation is performed. However, the present invention is not limited to this configuration. For example, the standard may be a single point numerical value rather than a numerical range, and also may be changed appropriately in accordance with the growth stage or the like. For example, an example in which the standard is a single point numerical value rather than a numerical range is realized as follows. In the first and second embodiments, the above-described standard range A1 (ppm) to A2 (ppm) for calcium ion is changed such that A1 becomes equal to A2, the above-described standard range B1 (ppm) to B2 (ppm) for magnesium ion is changed such that B1 becomes equal to B2, and the above-described standard range C1 (ppm) to C2 (ppm) for sodium ion is changed such that C1 becomes equal to C2. In addition, in the second embodiment, both the standard (standard value) and the target value for calcium ion are set to A1, both the standard (standard value) and the target value for magnesium ion are set to B1, and both the standard (standard value) and the target value for sodium ion are set to C1.

In the above-described embodiment, in the case where the control apparatus 12 performs instruction issuance or operation for adding two or more kinds of minerals to the rearing water W, the control apparatus 12 calculates the addition amounts of the two or more kinds of minerals to be added, mixes all the minerals in accordance with the calculated addition amounts, and adds the resultant mixture. However, in addition to the addition amounts, the control apparatus 12 may calculate their mixing ratio and their amounts by which the minerals are added each time when the minerals are added dividedly in a plurality of times. This is because, depending on the insufficient component(s) and the rearing environment, there is a case where it is desired to suppress the changing speeds of the concentrations of the minerals contained in the rearing water W by supplying the insufficient components stepwise while adjusting their mixing ratio, rather than supplying all the insufficient components at a time. This calculation may be performed in the case where a plurality of kinds of minerals are added together with water in step S32.

In the above-described embodiment, in the case where the control apparatus 12 performs instruction issuance or operation for adding water and minerals to the rearing water W, it is desired that the addition of water and the addition of the minerals are performed in the same period. However, in view of easiness of dissolution of the minerals into the rearing water and the work efficiency of the operator, a slight time lag may be provided so long as the cultured aquatic organism is not adversely affected.

The control apparatus 12 may produce a piece of information representing the relation between the amount of each mineral added to the rearing water (for example, the addition amount of each mineral per unit time) and a change over time in the concentration of this mineral in the rearing water. The control apparatus 12 may produce a piece of information representing the relation between the amount of water added to the rearing water (for example, the addition amount of water per unit time) and the concentration of each mineral in the rearing water. In the following description, a piece of information representing a change in mineral concentration when one or both of water and the mineral are added to the rearing water may be referred to as "concentration response information." The control apparatus 12 may produce the concentration response information by, for example, establishing the relation between the time history of the amount of a mineral added to the rearing water and the change over time in the concentration of this mineral. The time history of the amount of the mineral added to the rearing water is obtained, for example, from instructions issued when the control apparatus 12 controls the supply sections for the respective minerals. The change over time in the mineral concentration is obtained from the results of the detections by the mineral sensors. The control apparatus 12 learns, by means of machine learning, for example, a change over time in mineral concentration from the time when addition of the mineral to the rearing water is started. The control apparatus 12 may control the supply sections for the respective minerals by utilizing the result of the learning in such a manner, for example, as to reduce overshoot of concentration. The control apparatus 12 may update, for example, a function (for example, a mathematical expression or a computation table) used for determining the supply amount of each mineral, by using the result of the learning.

The control apparatus 12 reads out a program stored in, for example, the storage section 16 and executes various types of processes in accordance with this program. For example, the above-described program causes a computer to compare a predetermined standard and the concentration of each mineral which is contained in the rearing water within the rearing tank for rearing an aquatic organism or the rearing water within the circulation passage through which the rearing water circulates outside the rearing tank. In the case where the concentration of the mineral deviates from the standard, the above-described program may cause the computer to execute control for performing instruction issuance or operation for causing the mineral concentration of the rearing water to fall inside the standard. In the case where instruction issuance or operation for adding two or more kinds of minerals to the rearing water is performed, the above-described program may cause the computer to calculate the supply amounts of the two or more kinds of minerals to be added. The above-described program may cause the computer to execute control for performing instruction issuance or operation for adding to the rearing water the two or more kinds of minerals mixed together on the basis of the supply amounts. Before the instruction issuance or operation for adding water to the rearing water is performed, the above-described program may cause the computer to calculate the necessary water supply amount and to predict changes in the concentrations of the plurality of kinds of minerals as a result of addition of water of the calculated water supply amount. Further, when the concentration of at least one of the plurality of kinds of minerals is predicted to become lower than its standard, the above-described program may cause the computer to calculate the mineral supply amount necessary to render the concentration of the at least one kind of mineral coincident with the standard. The above-described program may cause the computer to execute control for performing instruction issuance or operation for adding water on the basis of the calculated water supply amount and control for performing instruction issuance or operation for adding the at least one kind of mineral on the basis of the mineral supply amount. In the case where it is predicted that none of the plurality of kinds of minerals becomes lower in concentration than the standard, the above-described program may cause the computer to execute control for performing instruction issuance or operation for adding water on the basis of the calculated water supply amount. The above-described program may be programed such that the computer does not execute part of the above-described various types of processes. The above-described program may be provided in a state in which the program is recorded on a computer readable storage medium. The above-described program may be a differential program or a differential file which executes the above-described various types of processes in cooperation with the program (for example, operating system) recorded in the computer system.

Notably, the minerals may be elements which are part of inorganic elements other than four elements (carbon, hydrogen, nitrogen, and oxygen) contained in ordinary organic matters and which affect growth of aquatic organisms. Namely, the minerals in the present invention are not limited to calcium, magnesium, and sodium and may be potassium, etc.

Notably, the embodiments disclosed this time should be considered to be illustrative and not to be restrictive in all aspects. The scope of the present invention is not limited to the embodiments disclosed this time, and it is intended that the present invention encompasses all modifications within the range shown by the claims and the range of equivalents of the claims.

### REFERENCE SIGNS LIST

- 12:: control apparatus (adjustment section)
- 22:: calcium sensor (mineral sensor)
- 24:: magnesium sensor (mineral sensor)
- 26:: sodium sensor (mineral sensor)
- 50:: rearing tank
- 70:: circulation passage
- Sy:: aquaculture system
- W:: rearing water

## Claims

1. An aquaculture system of a recirculating type for culturing an aquatic organism, comprising:
a mineral sensor for detecting the concentration of a mineral which is contained in rearing water within a rearing tank for rearing the aquatic organism or the rearing water within a circulation passage through which the rearing water circulates outside the rearing tank; and
an adjustment section which compares the concentration of the mineral detected by the mineral sensor with a predetermined standard and performs instruction issuance or operation for rendering the concentration of the mineral contained in the rearing water coincident with the standard when the mineral concentration deviates from the standard.

2. An aquaculture system according to claim 1, wherein the adjustment section performs instruction issuance or operation for adding the mineral to the rearing water when the mineral concentration detected by the mineral sensor is lower than the standard.

3. An aquaculture system according to claim 1 or 2, wherein the adjustment section performs instruction issuance or operation for adding water to the rearing water when the mineral concentration detected by the mineral sensor is higher than the standard.

4. An aquaculture system according to any one of claims 1 to 3, wherein the adjustment section performs operation for issuing a warning to the outside when the mineral concentration detected by the mineral sensor deviates from the standard.

5. An aquaculture system according to any one of claims 1 to 4, wherein the aquatic organism is a crustacean.

6. An aquaculture system according to any one of claims 1 to 5, wherein the mineral sensor is a liquid membrane type mineral sensor.

7. An aquaculture system according to any one of claims 1 to 6, wherein
the aquaculture system comprises mineral sensors for detecting the concentrations of a plurality of kinds of minerals; and
the adjustment section performs instruction issuance or operation for rendering the respective concentrations of the plurality of kinds of minerals coincident with their standards.

8. An aquaculture system according to claim 7, wherein, in the case where the adjustment section performs instruction issuance or operation for adding two or more kinds of minerals to the rearing water, the adjustment section calculates respective addition amounts of the two or more kinds of minerals to be added and performs instruction issuance or operation for adding to the rearing water the two or more kinds of minerals mixed together on the basis of the respective addition amounts.

9. An aquaculture system according to claim 7 or 8, wherein, in the case where the adjustment section performs instruction issuance or operation for adding water to the rearing water, before performance of the instruction issuance or operation for adding water to the rearing water, the adjustment section calculates a necessary water supply amount and predicts changes in the concentrations of the plurality of kinds of minerals as a result of addition of water of the water supply amount,
wherein, when the concentration of at least one of the plurality of kinds of minerals is predicted to become lower than its standard, the adjustment section calculates a mineral supply amount necessary to bring the concentration of the at least one kind of mineral to the standard and performs instruction issuance or operation for adding water on the basis of the calculated water supply amount and instruction issuance or operation for adding the at least one kind of mineral on the basis of the mineral supply amount, and
wherein, when it is predicted that none of the plurality of kinds of minerals becomes lower in concentration than the standard, the adjustment section performs instruction issuance or operation for adding water on the basis of the calculated water supply amount.
